# EUROPEAN PATENT SPECIFICATION

(11) **EP 2 649 161 B1**
(45) Date of publication and mention of the grant of the patent: **30.05.2018**
(21) Application number: 11805893.2
(22) Date of filing: 09.12.2011
(51) Int. Cl.: C10G 9/00, C10G 69/12, C10G 11/18, C10G 50/00

(54) **A METHOD FOR PRODUCTION OF MIDDLE DISTILLATE COMPONENTS FROM GASOLINE COMPONENTS BY OLIGOMERIZATION OF OLEFINS**
VERFAHREN ZUR HERSTELLUNG VON MITTELDESTILLATBESTANDTEILEN AUS BENZINBESTANDTEILEN DURCH OLIGOMERISIERUNG VON OLEFINEN
PROCÉDÉ DE PRODUCTION DE COMPOSÉS DE DISTILLAT MOYEN À PARTIR DE COMPOSÉS D'ESSENCE PAR OLIGOMÉRISATION D'OLEFINES

(30) Priority: 10.12.2010 FI 20106312
(43) Date of publication of application: 16.10.2013
(73) Proprietor: Neste Oyj, 02150 Espoo (FI)
(72) Inventor: MAKKONEN, Jaana, FI-01150 Söderkulla (FI); TIITTA, Marja, FI-06150 Porvoo (FI); MARKKANEN, Varpu, FI-01230 Vantaa (FI)
(74) Representative: Espatent Oy
(86) International application number: PCT/FI2011/051092
(87) International publication number: WO 2012/076758

(56) References cited:
- EP-A1- 1 602 705
- EP-A1- 2 333 031
- WO-A1-2011/135206
- US-A1- 2010 137 668
- US-B1- 6 613 108
- US-B1- 6 660 898
- E KRIVAN ET AL: "INVESTIGATION OF THE OLIGOMERIZATION OF LIGHT OLEFINS ON ION EXCHANGE RESIN CATALYST", HUNGARIAN JOURNAL OF INDUSTRIAL CHEMISTRY, [Online] vol. 38, no. 1, 1 January 2010 (2010-01-01), pages 53-57, XP55034096, ISSN: 0133-0276 Retrieved from the Internet: URL:http://konyvtar.uni-pannon.hu/hjic/HJI C38_053_057.pdf> [retrieved on 2012-07-27]

## Description

The present invention relates to petroleum refining and selective production of middle distillate fuel components wherein specific liquid blend compositions suitable for use as gasoline components are converted into middle distillate components suitable for aviation and diesel fuel.

### Background

The economics of fuel consumption tends to direct the choices between using diesel and gasoline fuel. Furthermore, the desire to lower carbon dioxide emissions in the end use is one important reason for increasing diesel production instead of gasoline production. The demand for middle distillates has been steadily rising in Western Europe. Occasionally, the demand for diesel has overcome that of gasoline creating a temporary need to be able to switch the production between these two. One shortcoming may be too low diesel supply, and another is increased gasoline production. The varying requirements due to demand changes have been balanced by trade flows between Europe, Russia and USA.
It is anticipated that the future refineries will have to invest in conversion processes that occasionally are able to increase the proportional yield of middle distillates.
As the carbon chain length of gasoline fuel components is from about C₄ to C₁₀ and for diesel fuel components from about C₁₀ to C₂₁ it is possible to increase the carbon chain length starting from gasoline components by dimerization or oligomerization. Most intensive studies for oligomerization or dimerization processes have typically involved lighter alkenes, such as C₄ olefins, due to their availability and use for gasoline components.
Oligomerisation reactions are defined as those which yield substances composed of molecules containing a few of one or more species of atoms or groups of atoms which are repetitively linked to each other. Catalysts used to oligomerize olefins are acid catalysts, such as zeolites, and non-zeolitic acid catalysts, nickel catalysts and transition metal catalysts. Heterogeneous acid catalysts, such as ZMS-5 and the like, have been found particularly suitable for the production of liquid fuels ranging from gasoline to diesel. Oligomerization reactions are not elementary, and are often accompanied by various parallel reactions such as cracking and isomerization. Thus, the outcome of an oligomerization process is not always predictable.

According to literature, both homogeneous and heterogeneous catalysts have been tested for higher alkenes oligomerization. Traditionally, sulfuric acid is applied and presently for example phosphoric acid impregnated on silica is in use. The best catalysts for oligomerization of higher alkenes, such as pentenes or hexenes, described in the literature are zeolites and mesoporous aluminosilicates due to their chemical stability and robustness in use. Their good performance is based on the high acidity of the catalysts which is essential to the oligomerization process. In these catalysts oligomerization reactions proceed on the large external surface areas or in mesopores of the zeolites. Oligomerisation is usually performed at temperatures above 200°C and at pressures above 50 bars (5000kPa). The main issue in use is associated to deactivation of the catalyst due to e.g. coke formation.

Attention should especially be paid towards yield and purity of dimers obtained, and to the operational life of the catalysts. Catalyst deactivation is a typical problem usually caused by pore blockage with heavy oligomers. Deactivation rate can be controlled by careful choice for appropriate catalyst, solvent and temperature combinations. Especially, the choice of right solvent has been found to decrease the deactivation rate of the catalysts. Good selectivity for producing desired dimers and trimers is required to enhance the process efficiency. At higher oligomerization temperatures the probability of side reactions tends to increase. Furthermore, the high reaction temperature causes aromatics formation. The content of aromatics in fuels is generally subject to regulatory limitations.

Only a few studies analyze the oligomerization products produced. Mostly, only dimers, trimers and tetramers have been reported, and no attention has been paid to other products such as aromatics and alkanes. Yet, these are important components when industrial processing is considered. Many commercial oligomerization processes are claimed to be suitable for a wide range of feedstock but the actual examples or analysis results for higher alkanes are not readily available.

There are several publications on oligomerizations using ZSM-5 type medium pore crystalline zeolite catalysts. For example, US4720600 discloses oligomerization of feedstock consisting essentially of C₃-C₆ mono-olefins, with varying amounts of nonreactive paraffins and the like acceptable components. The feedstock was contacted with medium pore acidic crystalline ZSM-type aluminosilicate catalyst having a silica to alumina molar ratio of at least 12, a constraint index of about 1-12 and acid cracking activity of about 160-200 for oligomerization at conditions to maximize distillate range products (C₁₀+). Optimum jet fuel distillate hydrocarbons were produced after oligomerization-polymerization and fractionating to the C₉-C₁₆ range of linear aliphatic hydrocarbons corresponding to a normal boiling point of about 165-290°C. US 6660898 B1 discloses a process for dimerizing light olefinic hydrocarbons in a reaction zone containing a solid acidic catalyst.

US 2010/0137668 A1 relates to a process for oligomerizing isoolefins.

E. Krivan et al. describe the oligomerization of light olefins on ion exchange resin catalyst (Hungarian Journal of Industrial Chemistry Veszprem, Vol. 38(1), pp. 53-57, 2010).

WO 2011/135206 A1 relates to a method for the oligomerization of olefins using at least one organic catalyst having a high density of acid sites.

US 6613108 B1 discloses a method for preparation of iso-octane from isobutene containing hydrocarbon feed by dimerizing at least part of isobutene into dibutene and subsequently hydrogenating it into iso-octane which is an excellent gasoline component. Enhanced selectivity and good yield of iso-octane is obtained when isobutene feedstock together with water is introduced into a reactor and contacted therein with an acidic ion exchange resin to form the dimerized product. The dimerized product, dibutene, is recovered by subsequent distillation and optionally hydrogenated into the paraffinic iso-octane product. This method was not anticipated to be effective for straight chained hydrocarbons due to their low reactivity compared to short chained and branched isobutene.
The object of the present invention is to provide an efficient method for converting gasoline fuel components into middle distillate fuel components.
A further object of the present invention is to provide aviation and/or diesel fuel components from gasoline components with good yield and improved low temperature properties.
A yet further object of the present invention is to provide aviation and/or diesel fuel components from gasoline components selectively and with a low content of aromatic compounds and sulfur.

### Summary of the invention

The inventors have found a viable route to industrial scale conversion of gasoline fuel components into aviation, such as jet fuel, and/or diesel fuel compatible components and thereby creating more flexibility for the production process tailoring.

In the present invention the regular refinery feed blends containing gasoline components i.e. higher alkenes, such as pentenes, hexenes and/or heptenes, together with alkanes are effectively oligomerized i.e. dimerized and/or trimerized, into middle distillate fuel components using ion-exchange resins as active and selective catalysts. This conversion is found to provide high quality aviation and/or diesel fuel components with good conversion yield, and the system can be operated for a long period of time before deterioration of the catalyst activity and capacity while performing the reaction at a low temperature.

The conversion process from gasoline fuel component fractions to aviation and/or diesel fuel components according to the present invention is provided in claim 1.

It was surprisingly found that an ion exchange resin catalyst, typically used in etherification process or e.g. for branched butene, is active enough in the dimerization and trimerization of C₅-C₁₀ olefins and capable of producing the desired products with high yield. Typically, alumina, silica or zeolite based catalysts are used for heptenes or hexenes instead of ion exchange catalyst due to anticipated activity problems and spatial incompatibility. In the method of the present invention only minor deactivation of the ion exchange catalyst was observed as a function of time during the oligomerization.

The present invention, moreover, enables the use of the same facilities, reactors and apparatus including the ion exchange catalyst that has been utilized in the manufacturing of ethers for gasoline components since the required reaction conditions, such as temperature and pressure ranges, are similar.

Compared to typically used silica, alumina and zeolite based catalysts, the present method operates essentially at lower reaction temperature. The deactivation rate of the ion exchange resin is dramatically lower than that of silica, alumina and zeolite based catalysts. This means longer operation times without the need for catalyst change or regeneration which renders the present method both economically and environmentally beneficial.

Furthermore, the catalyst deactivation rate is tunable. No poisonous additives such as alkylsulphide or alkylamines are required for feed.

### Figures

Figure 1a shows one schematic process configuration according to the present invention wherein dimerization or oligomerization reactors are located in parallel.
Figure 1b shows one schematic process configuration according to the present invention wherein dimerization or oligomerization reactors are located in series.
Figure 2 shows the amounts of dimers produced according to the method of the invention for example 1 and example 2.
Figure 3 shows the amounts of dimers and trimers produced according to the method of the invention for example 1 as a function of time.
Figure 4 shows the total conversion of olefins according to the method of the invention, example 1 compared to comparative examples 4 and 5.

### Detailed description of the invention

By the term "middle distillate" fuel component is meant fuel component having, substantially, carbon chain length of about C₁₀ or more, preferably from about C₁₀ to about C₂₁, which is a typical range for middle distillate suitable for use as diesel fuel or aviation fuel components.

The feed in the process of the present invention is a hydrocarbon mixture containing olefins which is typically used as gasoline component. Typically, the feed is a mixture of hydrocarbons having a boiling point in the range from 150°C to 400°C. The feed may be for example distilled from materials such as crude oil, or the products of a catalytic cracking or hydrocracking. The total amount of olefins in the hydrocarbon feed to be oligomerized is at least 10 wt-%, preferably at least 20 wt-%, more preferably at least 35%, most preferably at least 40% such as 45%. The rest is typically paraffins, aromatics and naphtenes.

The hydrocarbon feed containing olefins is selected from the group of regular gasoline components. These gasoline components comprise linear or branched C₅-C₁₁-olefins. Especially, the hydrocarbon feed comprises dimerizable or oligomerizable components; C₅-olefins, preferably isopentene whereby substituted C₁₀-olefins are produced; C₆-olefins, preferably isohexene whereby substituted C₁₂-olefins are produced, C₇-olefins, preferably isoheptene whereby substituted C₁₄-olefins are produced. It is clear that several higher olefins can be present in the feed, whereby a great variety of components is produced. Moreover, branched chain olefins are more preferred than linear ones due to their higher reactivity.

In one embodiment the hydrocarbon feed comprises mixed olefins originating preferably from catalytic cracking unit after distillation, more preferable distillate containing C5- to C7-olefins.

Even those gasoline components that are not particularly valuable may be used as part of the feed typically originating from thermal cracking unit or thermal catalytic cracking unit, such as methylpentenes, methylhexenes and methylheptenes as well as n-pentenes, n-hexenes and n-heptenes.

In the first step of the method of the present invention a liquid hydrocarbon blend composition i.e. feed mixture, suitable for use as a gasoline component is provided to a reactor. This feed comprises 0-90% by weight of C₅, 5-95% by weight of C₆ and 0-30% by weight of C₇ hydrocarbons containing olefins. In addition, at least one oxygenate is included into the liquid blend composition feed.
In one embodiment of the present invention the hydrocarbon feed contains C₅-olefins selected from the group of linear and branched C₅-olefins or a mixture thereof. More preferred are branched olefins due to their higher reactivity. The olefins most preferably present in the feed mixture comprise linear 1-, 2- or 3-pentene, 2-methyl-1-butene, 2-methyl-2-butene, 3,3-dimethyl-1-butene and 3-methyl-1-butene.
In one embodiment the hydrocarbon feed contains C₆ -olefins selected from the group of linear and branched C₆-olefins or a mixture thereof. More preferred are branched olefins due to their higher reactivity. The olefins most preferably present in the feed mixture comprise 2-methyl-2-pentene, 3-methyl-2-pentene, 2-methyl-1-pentene, 3-methyl-1-pentene, 2,2-dimethyl-1-butene, 2,3-dimethyl-1-butene, 2,4-dimethyl-2-butene, 2,3-dimethyl-2-butene, 2,4-dimethyl-2-butene and linear n-hexene.
In one embodiment the hydrocarbon feed contains C₇ -olefins selected from the group of linear and branched C₇-olefins or a mixture thereof. More preferred are branched olefins due to their higher reactivity. The olefins most preferably present in the feed mixture comprise 3-methyl-1-hexene, 2-methyl-3-hexene, 3-ethyl-1-pentene, 4,4-dimethyl-2-pentene, 2,3-dimethyl-1-pentene, 4-methyl-1-hexene.4-methyl-2-hexene, 2,3-dimethyl-2-pentene, n-heptene and the like.
The hydrocarbon feed additionally comprises higher hydrocarbons. Preferably, C8, preferably less than 23% by weight, and/or C9, preferably less than 16% by weight, and/or C10, preferably less than 6% by weight of the hydrocarbon containing olefins. A larger amount of higher hydrocarbons may cause undesired side reactions. Furthermore, the amount of aromatics tends to increase if the amount of higher hydrocarbons in the feed is increased.
The hydrocarbon feed most preferably comprises C₅, C₆ and/or C₇ iso-olefins. The amount of desired iso-olefins is preferably 10 - 40 wt-%.
Preferably, the amount of linear C5 and/or C6 and/or C7 hydrocarbons, such as 1-pentenes and the like, is less than 20% by weight.

The liquid blend composition feed for producing middle distillate components may be selected from the product or feed streams readily available at the refinery plant due to their use or manufacture in gasoline production. These particular gasoline streams contain particular compositions of C₅-C₁₁-fractions such as the product streams from fluid bed catalytic cracking unit (FCC), for example C₅-C₇ fraction from FCC distillation, stream from thermal cracking unit (VB, visbreaker), stream from thermal catalytic cracking unit (TCC), stream from deep catalytic cracking unit (DCC), stream from residue catalytic cracking unit (RCC), and/or stream from ethylene cracking unit (EC) comprising C₅ and C₆ raffinates. FCC unit product stream is especially preferred, since the hydrocarbon fractions can be used as such, but are preferably used after removing the lighter (≤C₄) or heavier (≥C₁₂) fractions. The product streams from these units typically need to be distilled for obtaining the suitable C₅-C₇ fractions. Typically, the streams from these units need to be distilled in order to obtain suitable C₅-C₇ fractions. On the contrary, the feed stream of C₅-C₇ etherification unit (TAME) is suitable for use as such for oligomerization feed.

The distillates to be used as liquid blend composition feed are preferably selectively hydrogenated for removal of dienes.

In a preferred embodiment C₅-C₇ etherification unit feed (TAME) is used as the liquid blend composition feed to the reactor. Preferably this feed comprises 70 - 90% C5 hydrocarbons, preferably trans-2-pentene and 2-methyl-2-butene, 5-20% C6 hydrocarbons, preferably 2-methyl-2-pentenes and 3-methyl-2-pentenes, and less than 2.5% by weight, preferably less than 2.0%, more preferably less than 1.8% of lower hydrocarbons, and less than 10% by weight, preferably less than 9%, more preferably less than 8% of higher hydrocarbons. As an example of a preferred TAME feed, preferably used after removal of dienes which are known to deactivate many acid catalysts, the following composition is given:

| hydrocarbon Cx | main component | % by weight |
|---|---|---|
| C4 | trans-2-butene | 0.5-1.5 |
| C5 | iso-pentane | 35-41 |
| C5 | trans-2-pentene | 12-14 |
| C5 | 2-methyl-2-butene | 18-22 |
| C5 | 2-methyl-1-butene | 3-6 |
| C6 | 4-methyl-2-pentene | 0.5-2 |
| C6 | 2-methylpentane | 3-5 |

In another preferred embodiment the feed originates from catalytic cracking unit (FCC) stream, preferably comprising a distilled composition of C5-C7 hydrocarbons from FCC unit. The heavy FCC gasoline stream comprises after free fractionation 20-50%, preferably 35-50%, more preferably 40-50%, most preferably 45-50%, C₅ hydrocarbons; 20-90%, preferably 40-80%, C₆ hydrocarbons; 5-20%, preferably 5-15%, C₇ hydrocarbons; less than 5%, preferably less than 2 wt-%, C₈ hydrocarbons. As a further example of preferred heavy FCC feed, the following fractionation is presented:

| hydrocarbon Cx | % by weight |
|---|---|
| C5 | 20-50 |
| C6 | 40-80 |
| C7 | 5-15 |
| C8 | <5 |

In a yet preferred embodiment C₆ fraction distilled from the FCC gasoline stream is used as the liquid blend composition feed to the oligomerization reactor comprising 1-20%, preferably 1-15%, more preferably 1-10%, most preferably 1-5% C5 hydrocarbons; 80-90%, preferably about 85%, C6 hydrocarbons; 5-10%, preferably about 8% C7 hydrocarbons; and less than 0.2% by weight, preferably less than 0.1%, more preferably less than 0.05% of lower hydrocarbons; and less than 0.2% by weight, preferably less than 0.1%, more preferably less than 0.05% of higher hydrocarbons.

In a preferred embodiment gasoline stream from VB unit is used for the liquid blend composition feed to the oligomerization reactor comprising 15-25%, preferably about 18%, C₅ hydrocarbons; 12-20% C₆ hydrocarbons, preferably about 17%, C₇ hydrocarbons; and less than 2.5% by weight, preferably less than 2.0%, more preferably less than 1.8% of lower hydrocarbons; and less than 10% by weight, preferably less than 9%, more preferably less than 8% of higher hydrocarbons. The distillation is preferably tailored to be restricted essentially to C5-C6-fractions.

In one embodiment gasoline stream from a TCC unit is used for the liquid blend composition feed to the oligomerization reactor comprising 10-20%, preferably 15-18%, C₅ hydrocarbons; 10-29%, preferably 15-20%, C₆ hydrocarbons; and less than 2.5% by weight, preferably less than 2.0%, more preferably less than 1.8% of lower hydrocarbons; and less than 10% by weight, preferably less than 9%, more preferably less than 8% of higher hydrocarbons. The distillation is preferably tailored to C5-C6-fraction.
In a preferred embodiment gasoline stream from ethene cracking unit is used as the liquid blend composition feed to the oligomerization reactor, preferably after fractionation.
Furthermore, selected mixtures of isopentene, isohexene and/or isoheptene prepared from chemicals may be used as feed.
Typically, the liquid blend composition feed originating from a refinery gasoline stream contains higher olefins and paraffines in the range essentially from C₅ to C₁₁, preferably from C₅ to C₉, more preferably from C₅ to C₇. The amount of aromates is less than 20%. When only e.g. C₅ olefins, only C₆ olefins or only C₇ olefins are fed to the process, it is clear that the resulting product of the reactions between the olefins yield dimers. However, when the feed composition comprises several different types of olefins such as C₅, C₆ and C₇, in addition to dimerization, reactions between the different olefins are likely to take place. The word "dimer" is also used for these reaction products for simplicity, but it is to be understood that when varying lower olefins are present in the feed, the resulting reaction mixture typically contains some amount of mixed dimers such as C₁₁, C₁₃ or C₁₅ olefins.
In one embodiment the liquid blend composition feed has a boiling point in the range of 20°C to 150°C, more preferably from 25-120°C, most preferably from 30-100°C. The vapor pressure for feed, such as the TAME unit feed, is from 500 to 1600 kPa.
It is noted that e.g. dimerization of isobutene into a gasoline component is a totally different type of technology compared to manufacture of middle distillates from C₅ or C₆ hydrocarbons by dimerization or trimerisation.
The sulfur content of the liquid blend composition feed is preferably less than 2000 ppm, preferably less than 1000 ppm, most preferably less than 90 ppm. The nitrogen content is preferably less than 10 ppm, preferably less than 5 ppb.
The feed rate and residence time for the feed inside the oligomerization is determined by liquid hourly space velocity (LHSV). Preferably, LHSV is from 0.05 to 20 h⁻¹, more preferably from 0.1 to 5 h⁻¹, most preferably from 0.2 to 3 h⁻¹. Depending on conversion yield, the unreacted feed may be recirculated back.

In addition to the liquid composition feed, at least one oxygenate is added together with it into the reactor to assist with oligomerization reactions. In the reaction mixture the oxygenate may form in situ an alcohol when reacting with an alkene, e.g. pentanol is a likely product when using the feed composition rich in pentene and water as oxygenate. One advantage is that the corresponding synthesized alcohol has the same carbon chain length as the corresponding alkene precursor. As disclosed in e.g. US 4375576, oxygen containing molecules, such as water, increase the dimer selectivity in isobutene dimerization and thus decrease the selectivity of trimerizing or tetramerizing reactions when dimerizing isobutene in the presence of an ion-exchange resin catalyst. This is found to apply in the case of higher alkenes, as well.

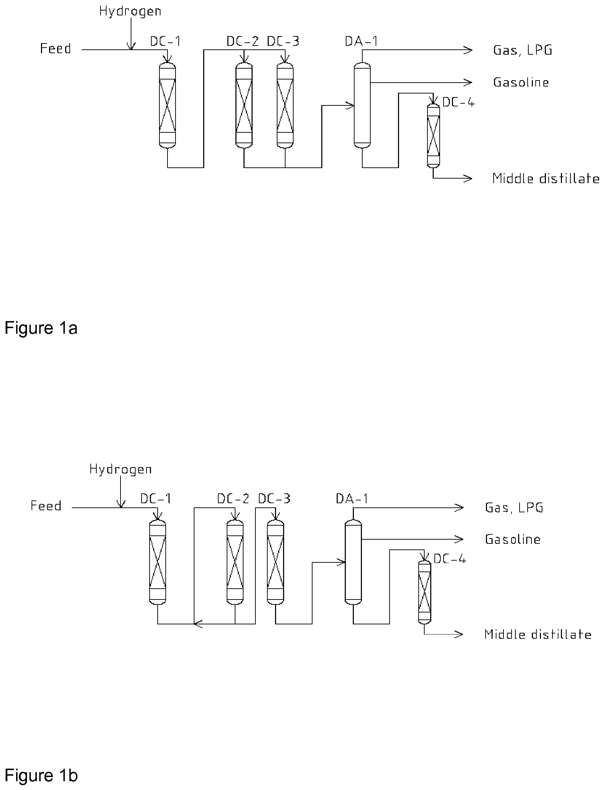

Presence of an oxygenate in oligomerization protects the catalyst in prolonged use. Moreover, oxygenate such as alcohol is able to hinder poisoning and the formation of larger molecules possibly blocking the catalyst and suppressing the activity. The oxygenate is preferably selected from the group of water or alcohol. The alcohol may be primary, secondary or ternary alcohol. Preferably the alcohol is C1-C6 alcohol, more preferably methanol, ethanol, isopropanol or t-butanol. The use of alcohol, such as ethanol and butanol, which remain in liquid state at ambient (outdoor) conditions are especially preferred.
The amount of oxygenate added to the olefinic blend is preferably less than 0.25% by weight of the olefins. The ratio of total oxygenate to total olefin content is from 0.001 to 0.7. Preferably, the oxygenate is added into the feed vessel or the feed is introduced to the saturator containing oxygenate. Alternatively, oxygenate may be added after the selective removal of dienes.
In one embodiment the selective removal of dienes is performed in reactor DC-1 as showed in figures 1a and 1b. The possible excess hydrogen is preferably removed before oligomerization.
In the second, oligomerization step ii, the liquid blend composition feed is contacted with an ion exchange resin catalyst, preferably a heterogeneous acidic ion exchange resin catalyst. The temperature of the feed is elevated into a range from 30°C to 200°C, preferably from 50°C to 150°C, more preferably from 70°C to 120°C, depending on the feed composition. The typical ion exchange resin type of catalyst will not withstand temperatures over 150°C without becoming thermally instable or starting to decompose. However, there are special, novel ion exchange resin catalysts that may hold even up to 200°C. In the reactor wherein the oligomerisation takes place the temperature and pressure are determined by the requirement for liquid phase reaction i.e. by feeding liquid composition into the reactor, the vapour pressure of the hydrocarbon constituents and the amounts thereof. Reaction in liquid phase decreases the formation and amount of intermediates or byproducts. The pressure in the oligomerisation step is adjusted within the range of from 500 to 2000 kPa, preferably from 900 to 1500 kPa, such as from 900 to 1100 kPa, depending on the temperature. The oligomerisation may take place in one or more reactors.
In one embodiment the oligomerization reactors, DC-2 and DC-3, are located in parallel as shown in figure 1a.
In another embodiment DC-2 and DC-3 of figure 1a can be used as separate oligomerization reactors or part of the feed can be led to DC-3.
In a yet further embodiment the oligomerization reactors, DC-2 and DC-3, are located in series as expressed in figure 1b. The outcoming stream of DC-2 or part of it can be circulated back as feed to DC-2.
It is important that the feed and the product are both at the same liquid state. This reduces the need for using high pressures which is preferred in industrial operation scale.
One major advantage in industrial scale is the possibility to use lower oligomerisation temperatures. Less energy is required for heating and it is even possible to use for example superheated steam for heating the mixture instead of furnaces. Lower operation temperatures are furthermore beneficial for reaction control.
Preferably, the heterogeneous acidic ion exchange resin catalyst to be used in the present invention is an acidic cationic polymer catalyst, more preferably strongly acidic sulfocation catalyst, most preferably a catalyst comprising styrene divinylbenzene copolymers with sulfuric acid type functional group(s), such as ion exchange catalysts like "Amberlyst-35 wet" with a COE of 5.2 (CAS 9049-93-8). The preferred catalyst has an acid concentration from 1 to 8 H⁺ eq/kg dry material. The acid concentration is preferably more than 3 H⁺eq/kg, more preferably more than 4 H⁺eq/kg, most preferably more than 4.5 H⁺eq/kg, such as over 5 H⁺eq/kg.

Activity of ion exchange resin catalysts in general depends on the acidity and accessibility of the acid sites. Number of acid sites is determined by the degree of sulfonation during preparation. Sulfonic acid group (-SO₃H) can be located on the macropore walls or within gel phase. The acid sites on the macropore walls are easily accessible. Accessibility of the acid sites in gel phase depends on the degree of crosslinking and polarity of the reaction medium. Strength of the acid sites varies depending on the degree of sulfurisation, the location of sulfonic acid group and the presence of polar components. More preferably, ion exchange resin catalyst used in the present invention has H+ -concentration of 2 - 8 H⁺eq/kg dry material, sulfur content of 15 - 20 wt-% and surface area (BET) from 1 - 80 m²/g. The particle size may vary but is typically about 0.1-0.5 mm.

The conversion of olefins of the liquid blend composition feed to dimers and trimers is preferably more than 80%, more preferably more than 90%.

In the third step iii, the resulting mixture containing still possibly partly unreacted feed components and oligomerized components is subjected to distillation for purification and separation of the oligomerized, preferably di- and/or trimerized, components. The unreacted feed components may be recycled back to the oligomerization reactor infeed.

In one embodiment the reacted liquid blend composition is led into a distillation column, such as DA-1 as shown in figures 1a and 1b, for removal of oligomerized components.

The selectivity of the oligomerization reactions i.e. dimerization and trimerization in a process according to present invention is high. According to one embodiment, the selectivity of oligomerized olefins, expressed as the ratio of the molar amount of dimeric and trimeric compounds to the total molar amount of converted olefins, is in excess of 0.8, preferably in excess of 0.9. Especially, the formation of dimers is clearly higher compared to oligomerization using other types of catalysts.

The heterogeneous acidic ion exchange catalyst of the present invention shows good activity judged by the obtained yield. However, even if the conversion is high, such as 90% or over, commercially a long operational life of the catalyst is even more important.

In the present invention the deactivation rate of the heterogeneous acidic ion exchange catalyst is much slower than when using other types of catalysts, preferably less than two percent units per 100 hours for 1-branced olefins, due to liquid composition feed rate and the amount and quality of impurities forming. It is noted that LHSV, oligomerization temperature and pressure are lower when using heterogeneous acidic ion exchange catalysts compared to more traditionally used alumina, silica or zeolite based catalysts. Typically, the dimer share to trimer share increases sharply (exponentially) due to trimers blocking the catalyst pores. This share increased uniformely with a linear less steep slope when using the heterogeneous acidic ion exchange resin catalyst of the present invention.

In the fourth step iv, the distillate i.e. the desired oligomerized fraction is hydrogenated, for example as shown in figures 1a and 1b in DC-4, after fractionation. In the hydrogenation the distillate is treated with hydrogen at an elevated pressure in the presence of a hydrogenation catalyst which is within the ordinary skill of an artisan in the field of hydrogenation.
According to one embodiment trimerization yield is enhanced by recycling at least partly the dimers obtained from step ii back to the feed of step i.
According to another embodiment trimerization yield is enhanced by dividing the feed to parallel reactors.
According to yet another embodiment trimerization yield is enhanced by dividing the feed to second and third reactor to have monomers to react with the dimers.
A high grade aviation fuel and diesel fuel component is thus obtained with good yield and selectivity. Further desired characteristics for the obtained high grade fuel are excellent cold properties, low sulfur content and low aromatic compound content. Moreover, especially desirable for a high grade diesel fuel component is an adequate density.
The product obtained by the method of the present invention has a high selectivity to dimers and trimers, the combined yield thereof being preferably more than 90%, more preferably more than 99%. Furthermore, conversion of olefins is more than 80%, more preferably more than 90%. The product obtained has excellent cold properties, preferably cloud point (determined according to ASTM D 5771) is below -25°C and pour point (determined according to ASTM D 5950) is below -70°C.
The middle distillate component produced by the present method is well suited for diesel blending based on the obtained hydrocarbon fractionation composition or as aviation, preferably jet fuel component due to accumulated lighter diesel fractions.

The good yield and selectivity achieved make the production clearly more efficient compared to previously used combination of processes and catalysts. Especially the long life time of the catalyst before deactivation rewards in increased production hours.

According to a preferred embodiment gasoline fractions of FCC unit, comprising 0-90% by weight of C₅, 5-95% by weight of C₆, 0-30% by weight of C₇ hydrocarbons containing olefins; less than 5% C₈ hydrocarbons; are converted to middle distillate components as presented in figure 1a. The hydrocarbon liquid blend composition feed is directed into column DC-1 together with oxygenate and additional hydrogen. Dienes are removed from the blend therein and excess hydrogen is preferably removed before dimerization. Thus treated blend is directed into parallel oligomerization reactors DC-2 and DC-3 and contacted with an ion exchange resin catalyst therein at a temperature from 30°C to 200°C, preferably from 50°C to 150°C, and at a pressure from 500 to 2000 kPa, preferably from 900 to 1500 kPa. The oligomerized products from the reactor outlets are directed into distillation column DA-1 for removal of reacted oligomers and for separation of liquid petroleum gas (LPG) and gasoline components. The oligomeric product is hydrogenated in DC-4 into the desired middle distillate and collected there from.

According to another preferred embodiment gasoline fractions of FCC unit are converted to middle distillate components as presented in figure 1b. The hydrocarbon liquid blend composition feed is directed into column DC-1 together with oxygenate and additional hydrogen. Dienes are removed from the blend in DC-1 and after removal of excess hydrogen the resulting product is directed into oligomerization reactors DC-2 and DC-3 in series and contacted with an ion exchange resin catalyst therein at a temperature from 30°C to 200°C, preferably from 50°C to 150°C, and at a pressure from 500 to 2000 kPa, preferably from 900 to 1500 kPa. The oligomerized product from the DC-3 reactor outlet is directed into distillation column DA-1 for removal of reacted oligomers and for separation of liquid petroleum gas (LPG) and gasoline components. The oligomeric product is hydrogenated in DC-4 into the desired middle distillate and collected there from.

The invention will be further illustrated by the following examples without being restricted thereto.

### Examples

Test runs were made in a plug flow microreactor. Typical particle size of catalyst according to the invention, Amberlyst -35 wet (Rohm&Haas), was 0.15 - 0.3 mm. Proton concentration of catalyst was 5.2 eq/kg dry material, sulfur content about 19 wt-% and surface areas (BET) about 50 m²/g. Catalysts were pre-treated overnight slightly above the reaction temperatures in nitrogen atmosphere. Reaction products were analyzed on-line with GC once a day.

### Example 1-5

The conditions of each experiment are given in Table 1.

**Table 1. The conditions of experiments.**

| | Example 1 | Example 2 | Example 3 | Comparative example 4 | Comparative example 5 |
|---|---|---|---|---|---|
| Catalyst | Amberlyst-35 wet (Rohm& Haas) | Amberlyst-35 wet (Rohm &Haas) | Amberlyst-35, wet (Rohm &Haas) | Commercial mesoporous aluminium silicate, SiO2-28 (Sasol) | Commercial natural zeolite, Montmorillonite K10-G (Sud-Chemie) |
| Feed | Feed 1 | Feed 1 with 0.25 wt-% tertiary butyl alcohol | Feed 2 | Feed 1 | Feed 1 |
| Reaction temperature (°C) | 100 | 100-120 | 100-120 | 100-250 | 175 |
| Pressure (kPa) | 1100 | 1100 | 1100 | 4100 | 4100 |
| WHSV (h-1) | 1 | 1 | 1 | 2,5-5 | 2,5 |

Two different feeds, feed 1 and feed 2, containing different amounts of olefins were used. Feed 1 is the feed for commercial etherification unit (TAME) and feed 2 is the stream from the distillation of FCC-unit. Both feeds contained mainly C₅ and C₆ olefins and paraffins.

The hydrocarbon composition of feed 1 is given in table 2.

**Table 2. Compounds and their amounts of feed 1.**

| hydrocarbon | % by weight |
|---|---|
| I-C4 | 0,01 |
| I+1-C4= | 0,12 |
| N-C4 | 0,22 |
| TR-2-C4= | 0,87 |
| 2,2-DIME-C3 | 0,00 |
| CIS-2-C4= | 0,47 |
| 3-ME-1-C4= | 0,13 |
| I-C5 | 37,97 |
| 1-C5= | 0,80 |
| 2-ME-1-C4= | 4,13 |
| N-C5 | 4,83 |
| TR-2-C5= | 12,23 |
| 3,3-DIME-1-C4= | 0,09 |
| CIS-2-C5= | 4,07 |
| ME-SYKLO-C4 | 0,00 |
| 2-ME-2-C4= | 20,07 |
| 2,2-DIME-C4 | 0,07 |
| SYKLO-C5= | 0,99 |
| 4-ME-1-C5= | 0,05 |
| 3-ME-1-C5= | 0,03 |
| SYKLO-C5 | 0,51 |
| 2,3-DIME-C4 | 1,31 |
| 2,3-DIME-1-C4= | 0,24 |
| CIS-4-ME-2-C5= | 0,15 |
| 2-ME-C5 | 4,42 |
| TR-4-ME-2-C5= | 0,51 |
| 3-ME-C5 | 1,99 |
| 2-ME-1-C5= | 0,37 |
| 2-ET-1-C4= | 0,00 |
| N-C6 | 0,31 |
| TR-3-C6=+CIS-3-C6= | 0,20 |
| TR-2-C6= | 0,42 |
| 2-ME-2-C5=+3-ME-SYKLO-C5= | 0,84 |
| CIS-3-ME-2-C5= | 0,34 |
| 4-ME-SYKLO-C5= | 0,01 |
| CIS-2-C6 | 0,15 |
| TR-3-ME-2-C5= | 0,42 |
| ME-SYKLO-C5 | 0,16 |
| 2,3-DIME-2-C4= | 0,16 |
| 1-ME-SYKLO-C5= | 0,22 |
| BENTGEENI | 0,10 |

The hydrocarbon composition of feed 2 is given in table 3. Feed 2 didn't contain any oxygenates.

**Table 3. Composition of feed 2.**

| Number of carbon atoms in hydrocarbon | Paraffins % by weight | Olefins % by weight | Naphtenes % by weight | Aromatics % by weight | Grand Total % by weight |
|---|---|---|---|---|---|
| 5 | 2,1 | 9,5 | 1,7 | 0,0 | 13,3 |
| 6 | 37,4 | 31,2 | 13,2 | 2,4 | 84,2 |
| 7 | 1,3 | 0,7 | 0,5 | 0,02 | 2,5 |
| Grand Total | 40,8 | 41,4 | 15,4 | 2,4 | 100 |

Impurities such as sulfur, nitrogen, water and oxygen compounds were analyzed for possible deactivating agents to the catalysts. Results are listed in table 4 for feed 1 and feed 2.

**Table 4. Amounts of different impurities in the feeds used in the tests.**

| Property | Test method | Unit | Feed 1 | Feed 2 |
|---|---|---|---|---|
| sulphur | EN ISO 20846 | % by weight | 0.0010 | 0.0088 |
| water | EN ISO 12937 | mg/kg | 32 | 30 |
| nitrogen | ASTM D 4629 (Antek) | µg/kg | <5 | <5 |
| oxygen compounds | Gas Cromatography | % by weight | 0.18 | 0.02 |

Subsequently, the dimerization/oligomerization product was fractionated by distillation for removal of higher, over C₁₀ fractions, and the obtained diesel fraction was hydrogenated in autoclave (Parr with Kataleuna KL5664-TL 1.2 mm) at 200°C and in 35 bar. This resulted in C₇₋₁₆ fractions as shown in table 5.

**Table 5. Amount of different hydrocarbons based on carbon number in distilled and hydrogenated product.**

| Number of carbon atoms in hydrocarbon | Amount in wt-% (GC) |
|---|---|
| 7 | 0.08 |
| 8 | 0.5 |
| 9 | 9.1 |
| 10 | 62.4 |
| 11 | 17.7 |
| 12 | 1.8 |
| 13 | 0.8 |
| 14 | 2.5 |
| 15 | 4.9 |
| 16 | 0.4 |

### Results

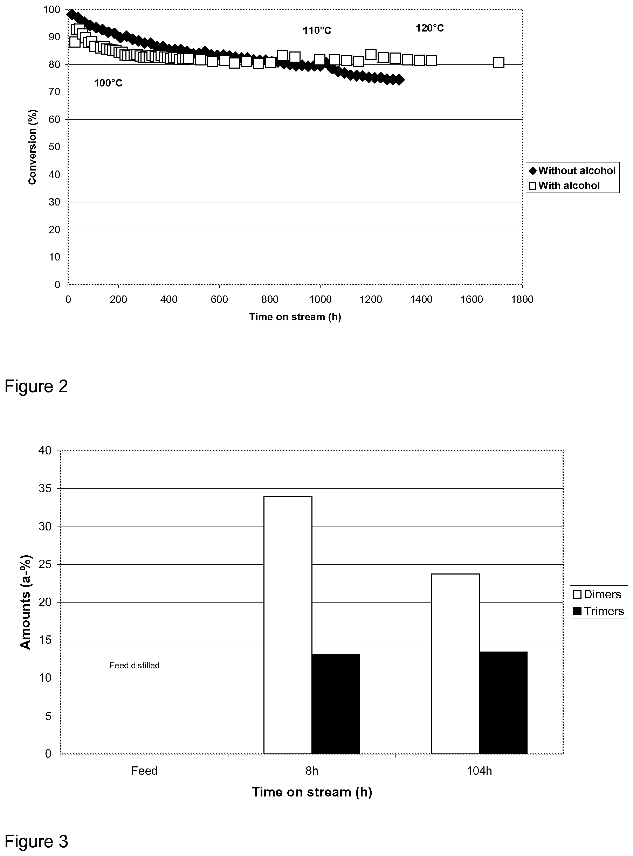

Figure 2 shows the obtained dimers as dependent of processing time for example 1 using TAME feed (curve named without alcohol) and for example 2 using TAME feed with oxygenate (curve named with alcohol). The used ion exchange catalyst, Amberlyst-35 wet, was found to exhibit an excellent activity and long period of use. Some decrease in dimer yield is obtained in prolonged use for example. This decrease could be suppressed by incorporation of temperature profile from 100 to 120°C and addition of oxygenate as shown for example 2. Virtually, a stable formation of dimers was achieved for the tested time frame in example 2. Figure 3 shows the amounts of dimers and trimers produced according to the method of the invention for example 1 as a function of time.

The properties of distilled and hydrogenated products of example 2 are given in Table 6.

**Table 6. Properties of two different middle distillate products made from experiment 2 products.**

| Method | | Distilled and hydrogenated product of example 2 (contains mainly dimers) | Distilled and hydrogenated product of example 2 (contains mainly trimers) |
|---|---|---|---|
| EN ISO 12185 +15°C | Density (kg/m3) | 776,8 | 814,3 |
| ASTM D 5771 | Cloud point (°C) | -62.3 | -30,5 |
| ASTM D 6890 | IQT cetane number | 27,7 | 25,5 |
| Distillation IBP | °C | 170,1 | 220,3 |
| 5% | °C | 176,2 | 225 |
| 50% | °C | 185,2 | 250,5 |
| 90% | °C | 208,6 | 309,9 |
| 95% | °C | 221,6 | 345,6 |
| EP | °C | 241,4 | 358,6 |

As shown by the table 6, the middle distillate products have excellent cold properties. The density enables the use of both fractions as components for the preparation of diesel fuel.

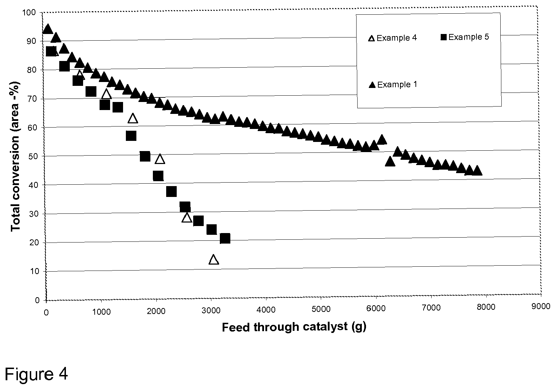

Figure 4 shows the differences in the catalyst deactivation behaviour for example 1 using ion exchange catalyst compared to comparative examples 4 (curve named example 4) and 5 (curve named example 5) using other commercial catalysts. The ion exchange resin catalyst Amberlyst-35 used results in the highest conversions and total amount of desired products compared to experiments using other commercial comparative catalysts. Furthermore, the need for catalyst regeneration is diminished.

## Claims

1. A method for producing middle distillate fuel components from gasoline fuel component fractions by oligomerization of olefins wherein the method comprises the steps of
i. providing a reactor with a liquid blend composition feed suitable for use as gasoline component comprising 0-90% by weight of C₅, 5-95% by weight of C₆, 0-30% by weight of C₇ hydrocarbons containing olefins, at least one oxygenate, the weight ratio of the total amount of said oxygenate to total olefin content being from 0.001 to 0.7, with hydrogen feed, and aromatics less than 20% by weight, and
ii. subjecting said feed to oligomerisation by contacting said feed with an ion exchange resin catalyst at a temperature from 30°C to 200°C, preferably from 50°C to 150°C, and at a pressure from 500 to 2000 kPa, preferably from 900 to 1500 kPa, and
iii. separating the obtained oligomerized feed of step ii from the unreacted feed by distillation, and
iv. hydrogenating said distillate obtained in step iii, for forming a product suitable for use as a middle distillate fuel component.

2. The method according to claim 1 wherein said feed comprises at least 20% by weight of olefins.

3. The method according to claims 1 or 2 **characterized in that** said catalyst is a heterogeneous acidic ion exchange resin catalyst, preferably an acidic cationic polymer catalyst, more preferably strongly acidic sulfocation catalyst, most preferably a catalyst comprising styrene divinylbenzene copolymers with sulfuric acid type functional group(s).

4. The method according to claim 3 **characterized in that** the acid concentration of said catalyst is from 1 to 8 eq/kg dry material.

5. The method according to any one of the previous claims **characterized in that** said olefins of C₅ hydrocarbons are selected from the group of 1-, 2- or 3-pentene, 2-methyl-1-butene, 2-methyl-2-butene, 3,3-dimethyl-1-butene and 3-methyl-1-butene.

6. The method according to any one of the previous claims **characterized in that** said olefins of C₆ hydrocarbons are selected from the group of 2-methyl-2-pentene, 3-methyl-2-pentene, 2-methyl-1-pentene, 3-methyl-1-pentene, 2,2-dimethyl-1-butene, 2,3-dimethyl-1-butene, 2,4-dimethyl-2-butene, 2,3-dimethyl-2-butene, 2,4-dimethyl-2-butene and linear n-hexene.

7. The method according to any one of the previous claims **characterized in that** said olefins of C₇ hydrocarbons are selected from the group of 3-methyl-1-hexene, 2-methyl-3-hexene, 3-ethyl-1-pentene, 4,4-dimethyl-2-pentene, 2,3-dimethyl-1-pentene, 4-methyl-1-hexene, 4-methyl-2-hexene, 2,3-dimethyl-2-pentene, n-heptene.

8. The method according to any one of the previous claims **characterized in that** said feed originates from catalytic cracking unit (FCC) stream, preferably comprising a distilled composition of C5-C7 hydrocarbons from FCC stream.

9. The method according to any one of the previous claims **characterized in that** said feed comprises the feed stream of C₅-C₇ fraction from etherification unit (TAME).

10. The method according to any one of the previous claims **characterized in that** said feed originates from thermal catalytic cracking unit (TCC) or thermal cracking unit (VB, visbreaker) stream.

11. The method according to any one of the previous claims **characterized in that** said feed originates from deep catalytic cracking unit (DCC) stream.

12. The method according to any one of the previous claims **characterized in that** said feed originates from residue catalytic cracking unit (RCC) stream.

13. The method according to any one of the previous claims **characterized in that** said feed originates from ethylene cracking unit (EC) stream, preferably from ethylene cracking unit (EC) stream comprising mainly C₅ and C₆ raffinates.

14. The method according to any one of the previous claims **characterized in that** dienes have been removed from said feed before providing it to step i.

## Patentansprüche

1. Verfahren zur Herstellung von Mitteldestillatkraftstoffkomponenten aus Benzinkraftstoffkomponentenfraktionen durch Oligomerisierung von Olefinen, wobei das Verfahren umfasst die Schritte von
i. Bereitstellen eines Reaktors mit einem Flüssigkeitsmischungszusammensetzungszufuhrmaterial geeignet zur Verwendung als Benzinkomponente umfassend 0-90 Gew.-% von C₅, 5-95 Gew.-% von C₆, 0-30 Gew.-% von C₇ Kohlenwasserstoffen, die Olefine enthalten, zumindest ein Oxygenat, das Gewichtsverhältnis der Gesamtmenge des Oxygenats zum Gesamtolefingehalt ist 0,001 bis 0,7, mit Wasserstoffzufuhrmaterial, und Aromaten weniger als 20 Gew.-%, und
ii. Unterwerfen des Zufuhrmaterials der Oligomerisierung durch Kontaktieren des Zufuhrmaterials mit einem Ionenaustauschharzkatalysator bei einer Temperatur von 30°C bis 200°C, bevorzugt von 50°C bis 150°C, und einem Druck von 500 bis 200 kPa, bevorzugt von 900 bis 1500 kPa, und
iii. Trennen des erhaltenen oligomerisierten Zufuhrmaterials von Schritt ii von dem unreagierten Zufuhrmaterial durch Destillation, und
iv. Hydrierung des in Schritt iii enthaltenen Destillats, zum Bilden eines Produkts geeignet zur Verwendung als eine Mitteldestillatkraftstoffkomponente.

2. Verfahren nach Anspruch 1, wobei das Zufuhrmaterial zumindest 20 Gew.-% an Olefinen umfasst.

3. Verfahren nach Anspruch 1 oder 2, **dadurch gekennzeichnet, dass** der Katalysator ist ein heterogener saurer Ionenaustauschharzkatalysator, bevorzugt ein saurer kationischer Polymerkatalysator, mehr bevorzugt stark saurer Sulfokationkatalysator, am meisten bevorzugt ein Katalysator umfassend Styroldivinylbenzol-Copolymere mit schwefelsäureartiger funktioneller Gruppe(n).

4. Verfahren nach Anspruch 3, **dadurch gekennzeichnet, dass** die Säurekonzentration des Katalysators von 1 bis 8 eq/kg Trockenmaterial ist.

5. Verfahren nach einem der vorstehenden Ansprüche, **dadurch gekennzeichnet, dass** die Olefine von C₅ Kohlenwasserstoffen ausgewählt sind aus der Gruppe von 1-, 2- oder 3-Penten, 2-Methyl-1-buten, 2-Methyl-2-buten, 3,3-Dimethyl-1-buten und 3-Methyl-1-buten.

6. Verfahren nach einem der vorstehenden Ansprüche, **dadurch gekennzeichnet, dass** die Olefine von C₆ Kohlenwasserstoffen ausgewählt sind aus der Gruppe von 2-Methyl-2-penten, 3-Methyl-2-penten, 2-Methyl-1-penten, 3-Methyl-1-penten, 2,2-Dimethyl-1-buten, 2,3-Dimethyl-1-buten, 2,4-Dimethyl-2-buten, 2,3-Dimethyl-2-buten, 2,4-Dimethyl-2-buten und linearem n-Hexen.

7. Verfahren nach einem der vorstehenden Ansprüche, **dadurch gekennzeichnet, dass** die Olefine von C₇ Kohlenwasserstoffen ausgewählt sind aus der Gruppe von 3-Methyl-1-hexen, 2-Methyl-3-hexen, 3-Ethyl-1-penten, 4,4-Dimethyl-2-penten, 2,3-Dimethyl-1-penten, 4-Methyl-1-hexen, 4-Methyl-2-hexen, 2,3-Dimethyl-2-penten, n-Hepten.

8. Verfahren nach einem der vorstehenden Ansprüche **dadurch gekennzeichnet, dass** das Zufuhrmaterial stammt aus einem Strom einer katalytischen Crackeinheit (FCC), bevorzugt umfassend eine destillierte Zusammensetzung von C5-C7 Kohlenwasserstoffen aus FCC-Strom.

9. Verfahren nach einem der vorstehenden Ansprüche, **dadurch gekennzeichnet, dass** das Zufuhrmaterial umfasst den Zufuhrmaterialstrom von C₅-C₇ Fraktion von Veretherungseinheit (TAME).

10. Verfahren nach einem der vorstehenden Ansprüche, **dadurch gekennzeichnet, dass** das Zufuhrmaterial stammt von einem Strom aus einer thermischen katalytischen Crackeinheit (TCC) oder thermischen Crackeinheit (VB, Visbreaker).

11. Verfahren nach einem der vorstehenden Ansprüche, **dadurch gekennzeichnet, dass** das Zufuhrmaterial stammt von einem Strom aus einer eingehenden katalytischer Crackeinheit (DCC) (deep catalytic cracking unit (DCC)).

12. Verfahren nach einem der vorstehenden Ansprüche **dadurch gekennzeichnet, dass** das Zufuhrmaterial stammt vom einem Strom aus einer katalytischen Rückstandscrackeinheit (RCC) (residue catalytic cracking unit (RCC)).

13. Verfahren nach einem der vorstehenden Ansprüche **dadurch gekennzeichnet, dass** das Zufuhrmaterial stammt von einem Strom aus einer Ethylencrackeinheit (EC), bevorzugt von einem Strom aus einer Ethylencrackeinheit (EC) umfassend hauptsächlich C₅ und C₆ Raffinate.

14. Verfahren nach einem der vorstehenden Ansprüche **dadurch gekennzeichnet, dass** Diene aus dem Zufuhrmaterial entfernt wurden, bevor dieses dem Schritt i bereitgestellt wurde.

## Revendications

1. Procédé de production de composants de carburant de distillat moyen à partir de fractions de composants de carburant de type essence par oligomérisation d'oléfines, dans lequel le procédé comprend les étapes de
i. fournir à un réacteur une charge d'une composition de mélange liquide appropriée pour une utilisation comme composant d'essence comprenant de 0 à 90% en poids de C₅, de 5 à 95% en poids de C₆, de 0 à 30% en poids d'hydrocarbures en C₇ contenant des oléfines, au moins un composé oxygéné, le rapport en poids de la quantité totale dudit composé oxygéné sur la teneur totale du contenu en oléfine étant compris entre 0,001 et 0,7, avec une charge d'hydrogène, et moins de 20% en poids d'aromatiques, et
ii. soumettre ladite charge à une oligomérisation en mettant en contact ladite charge avec un catalyseur à résine échangeuse d'ions à une température de 30°C à 200°C, de préférence de 50°C à 150°C et à une pression de 500 à 2000 kPa, de préférence de 900 à 1500 kPa, et
iii. séparer la charge oligomérisée obtenue à l'étape ii de la charge n'ayant pas réagi par distillation, et
iv. hydrogéner ledit distillat obtenu à l'étape iii, pour former un produit approprié pour une utilisation en tant que composant de carburant de distillat moyen.

2. Le procédé selon la revendication 1, dans lequel ladite charge comprend au moins 20% en poids d'oléfines.

3. Le procédé selon la revendication 1 ou 2, **caractérisé en ce que** ledit catalyseur est un catalyseur à résine échangeuse d'ions acide hétérogène, de préférence un catalyseur polymère cationique acide, plus préférablement un catalyseur sulfocationique fortement acide, le plus préférablement un catalyseur comprenant des copolymères de styrène divinylbenzène avec des groupes fonctionnels de type acide sulfurique.

4. Le procédé selon la revendication 3, **caractérisé en ce que** la concentration en acide dudit catalyseur est de 1 à 8 éq/kg de matière sèche.

5. Le procédé selon l'une quelconque des revendications précédentes, **caractérisé en ce que** lesdites oléfines d'hydrocarbures en C₅ sont choisies dans le groupe constitué de 1-, 2- ou 3-pentène, 2-méthyl-1-butène, 2-méthyl-2-butène, 3,3-diméthyl-1-butène et 3- méthyl-1-butène.

6. Le procédé selon l'une quelconque des revendications précédentes, **caractérisé en ce que** lesdites oléfines d'hydrocarbures en C₆ sont choisies dans le groupe constitué de 2-méthyl-2-pentène, 3-méthyl-2-pentène, 2-méthyl-1-pentène, 3-méthyl-1-pentène, 2,2-diméthyl-1-butène, 2,3-diméthyl-1-butène, 2,4-diméthyl-2-butène, 2,3-diméthyl-2-butène, 2,4-diméthyl-2-butène et n-hexène linéaire.

7. Le procédé selon l'une quelconque des revendications précédentes, **caractérisé en ce que** lesdites oléfines d'hydrocarbures en C₇ sont choisies dans le groupe constitué de 3-méthyl-1-hexène, 2-méthyl-3-hexène, 3-éthyl-1-pentène, 4,4-diméthyl-2-pentène, 2,3-diméthyl-1-pentène, 4-méthyl-1-hexène, 4-méthyl-2-hexène, 2,3-diméthyl-2-pentène, n-heptène.

8. Le procédé selon l'une quelconque des revendications précédentes, **caractérisé en ce que** ladite charge provient d'un flux d'une unité de craquage catalytique (FCC), comprenant de préférence une composition distillée d'hydrocarbures en C5-C7 provenant d'un flux de FCC.

9. Le procédé selon l'une quelconque des revendications précédentes, **caractérisé en ce que** ladite charge comprend le flux d'alimentation en fraction C₅-C₇ d'une unité d'éthérification (TAME).

10. Le procédé selon l'une quelconque des revendications précédentes, **caractérisé en ce que** ladite charge provient d'un flux d'une unité de craquage catalytique thermique (thermal catalytic cracking unit, TCC) ou d'une unité de craquage thermique (VB, visbreaker).

11. Le procédé selon l'une quelconque des revendications précédentes, **caractérisé en ce que** ladite charge provient d'un flux d'une unité de craquage catalytique profond (deep catalytic cracking unit, DCC).

12. Le procédé selon l'une quelconque des revendications précédentes, **caractérisé en ce que** ladite charge provient d'un flux d'une unité de craquage catalytique de résidu (residue catalytic cracking unit, RCC).

13. Le procédé selon l'une quelconque des revendications précédentes, **caractérisé en ce que** ladite charge provient d'un flux d'une unité de craquage d'éthylène (EC), de préférence d'un flux d'une unité de craquage d'éthylène (EC) comprenant principalement des raffinats en C₅ et C₆.

14. Procédé selon l'une quelconque des revendications précédentes, **caractérisé en ce que** des diènes ont été retirés de ladite charge avant de la fournir à l'étape i.
